# EUROPEAN PATENT APPLICATION

(11) **EP 2 417 968 A1**
(43) Date of publication of application: **15.02.2012**
(21) Application number: 10425256.4
(22) Date of filing: 29.07.2010
(51) Int. Cl.: A61K 9/16, A61K 9/51, A61K 38/20, A61K 38/21, A61K 39/395

(54) **Particle comprising cytokines, antibodies and polymers and use thereof as medicament for the treatment of cancer**

(71) Applicant: Consorzio per il Centro di Biomedicina Molecolare Scrl, 34012 Basovizza (TS) (IT)
(72) Inventor: Krol, Silke, 34012 Basovizza Trieste (IT)
(74) Representative: Spadaro, Marco

(57) **Abstract**

The present invention refers to a particle comprising cytokines with an antibody, connected to a polymer, which release the cytokines and the antibody in the extracellular environment of the tumor site, providing a guided and enhanced immune response. It also refers to said particle for use a medicament, in particular for use as drug delivery system and as an anti-cancer agent, to pharmaceutical composition and a process for the preparation thereof.

## Description

### Technical Field

The present invention relates to the medical field, in particular to the treatment of cancer.

### Background Art

Cytokines are transmitter molecules from the immune system. By their means, the immune cells can communicate to each other. Such a communication is important for the regulation and tuning of the complex immune response of the body. Cytokines are able to activate or inhibit the immune cells or induce their proliferation and production.

In principle three groups of cytokines can be distinguished: growth factors of the hematopoiesis, interleukins and interferons.

Since several years, the use of cytokines for treatment of tumors was discussed and a huge potential for future therapies is expected. But up to now the cytokine-based therapeutic approaches are rarely used because of unsolved severe drawbacks of the treatment. One problem is the fast clearance time of cytokines from the body and the dosage in therapy. Cytokines can be extremely toxic and they should be targeted against the tumors (e.g. J. Descotes, A. Gouraud Clinical immunotoxicity of therapeutic proteins. Expert Opinion on Drug Metabolism & Toxicology, r 2008, 4, 1537-1549.). The treatment with cytokines can have severe side-effects like fever, exhaustion, rash, tachycardia, increase of weight and allergic reaction leading to multiple organ failure. In immune therapy against cancer, cytokine are typically used as unspecific auxiliary substances to support other immune therapies, e.g. vaccination. Cytokines are also administered additionally to a chemotherapy (biochemotherapy), an antibody treatment or a gene therapy.

Cancer therapy is still a difficult task for the person expert in this field. Other than surgery, cancer therapy uses complex therapeutic protocols with anticancer drugs and combinations of drugs and/or radiotherapy. Cancer therapy is mostly affected by severe side effects. A solution provided by the present invention makes use of the natural defence system of the body, which is circumvented by the tumor cells, in well-balanced way and hence limiting the side effects.

The patent application WO 2005/55979 A2 discloses a drug delivery system comprising pH triggerable particles. The pH triggerable particles comprise an agent(s) to be delivered, which is encapsulated in a matrix comprising a pH trigger agent and a polymer. Agents including nucleic acids may be delivered intracellular using the inventive pH triggerable particles. Upon exposure to an acidic environment such as the endosome or phagosome of a cell, the particles dissolve or disrupt due to protonation or an increase in solubility of the pH triggering agent.

The patent application WO 03/063909 A1 discloses a responsive microgel which responds volumetrically and reversibly to a change in one or more aqueous conditions selected from temperature, pH and ionic conditions, comprised of an ionizable network of covalently cross-linked homopolymeric ionizable monomers wherein the ionizable network is covalently attached to an amphiphilic copolymer to form a plurality of 'dangling chains' and wherein the 'dangling chains' of amphiphilic copolymer form immobile micelle-like aggregates in aqueous solution. Particularly, the responsive microgel is able to imbibe or solubilize a large amount of therapeutic agent and deliver a substantially linear and sustained release of therapeutic agent under physiological conditions.

The patent application WO 2006/073950 A2 discloses the nanoparticles composed of chitosan, poly-y-glutamic acid, and at least one bioactive agent characterized with a positive surface charge and their enhanced permeability for paracellular drug delivery.

The patent application US 2009/0016962 A1 discloses nanostructures comprising a charged outer surface and an inner core comprising a cancer therapeutic agent or imaging agent, wherein the charged outer surface is selectively removable. Further provided are methods of treating subjects having cell proliferative disorders, e.g., cancer, and kits comprising the above nanostructures.

The patent application WO 2008/107729 A1 relates to a sustained drug release nanocomposite, comprising a) a charge carrying core of primary protein particles reversibly precipitated in an ionic procedure from solutions containing at least one cytokine drug and b) a poly-electrolyte shell comprised of 3 to 5 alternate layers of two oppositely charged polyelectrolytes, the dissociation state of one of the polyelectrolytes being varied with pH of the aqueous medium while the other polyelectrolyte is fully dissociated, irrespective of the pH of the medium. There is also provided a process for producing the said nanocomposite and the use of the same for producing sustained release drug formulations. None of these systems solves the technical problem of activating the immune system, normalizing the pH condition in the tumor environment, delivering cytokines and/or an antibody adequately protected to the tumor sites and triggered by the lower tumoral pH releasing them in the extracellular environment, providing a natural activation of the immune system and normalization of the conditions in the tumor to allow for an adequate tumor destruction by the immune system.

Therefore the need for a drug delivery system capable of specifically and selectively arriving to the tumor site, and capable of activating the immune system in the tumor site, without producing side effects is still felt.

The need of controlled release of a well defined dose of cytokines for tumor treatment is also felt.

### Disclosure of Invention

It has now been found that a particle comprising a low-dose cytokines with an antibody, connected to a polymer is able to arrive at the tumor site and, because of the lower pH, to release the cytokines and the antibody in the extracellular environment, providing a guided and enhanced immune response.

The polymer is utilized as a carrier material which protects the cytokines and antibodies during passage in the blood, releases the cytokines and antibodies under tumor specific conditions, regulates the tumor-specific pH to the physiologic one and helps to enhance the effective concentration of cytokines and antibodies within the tumor bed.

While releasing the cytokines, the polymer is able to adjust the pH of the cancer tissue to 7.4 (the physiological value), thus enables the immune system in a physiological microenvironment to respond to the tumor.

Therefore, it is an object of the present invention a particle comprising at least one immune-activating cytokine, at least one antibody against an immune-suppressor cytokine and a polymer with a pK between 6.5 and 7.4 or a pharmaceutically acceptable salt thereof, the above particle releases said cytokine and said antibody in the acidic environment of a solid tumor.

In a preferred embodiment the polymer is selected from the group consisting of: chitosan and its derivatives, poly-(acrylic acid-*co*-maleic acid) (PAA-MA), also known as poly-(acrylamide-*co*-maleic acid), poly(4-styrenesulfonic acid-*co-*maleic acid) sodium salt and quaternized chitosan.

In a preferred embodiment, said immune-activating cytokine is selected from the group consisting of: IL-2, IL-4 and IFN-γ.

In another preferred embodiment said antibody is Anti-IL-10.

A further object of the present invention is the above particle for use as medicament, in particular for use as drug delivery system, wherein said drug is at least one immune-activating cytokine and at least one antibody against an immune-suppressor cytokine.

In a preferred embodiment said drug is selectively delivered to the acidic tumor site.

An object of the present invention is the above particle for use as anti-cancer agent.

Further objects of the invention are a pharmaceutical composition comprising an effective amount of the above particle and a process for the preparation of said particle.

Pharmaceutical compositions are known and can be prepared with conventional processes, for example as described in Remington's Pharmaceutical Sciences, last edition, Mack Publishing and Co.

A further object of the present invention is a method for treating cancer by administering the above particles to a patient in need thereof. In particular, the administration comprises an affective amount of said particles.

These and other objects of the present invention will now be illustrated in detail also by means of examples.

### Modes for Carrying Out the Invention

In this context, the term "polymer" has the same meaning of the term "polyelectrolyte".

According to the present invention, the polymer has different functions in this context and provides different advantages:
- it stabilizes the cytokine by preventing or reducing the degradation by proteases and prevents inhibition by antibodies;
- it prevents severe side-effects by antibody therapy;
- it provides a pH-induced release of the cytokine;
- the cytokine or the antibody is released if the polyelectrolyte interacts with the protons from the more acidic environment of the tumor (pH 6.5 vs. 7.4 in healthy tissue). Due to this targeted and controlled release high local concentrations of the cytokine can be reached while the overall concentration in the body could be extremely low;
- the proton-binding capacity of the polyelectrolyte causes an increase of the pH in the tumor which leads to an improved immune response of the body; the immune response is optimized to work at the normal pH of 7.4 and is not fully effective at lower pH values.

The polyelectrolyte is now slightly positively charged (polycation) and can bind to the tumor cell surface which is known to be more negative than that of normal cells and guide the immune response against the marked tissue. The biodegradation is slow so the polycation will bind and not degrade to monomers after delivering the cytokine or antibody. Differently from prior art, the particles of the present invention can mark the tumor cells, by binding to tumor cell surface. The delayed biodegradation is important from the point of view of controlled release of the active agents, cytokines, antibody and optionally other antitumor agents, and the regulatory requirements for approval of the particles of the present invention for administration to humans and animals The polyelectrolyte according to the present invention shows the following characteristics:
- pK between 6.5 and 7.4;
- not immediately biodegradable;
- weak binding between polymer and cytokine/antibody. Said weak binding is due to the fact that the polymer has a low charge density.

The polymer of the invention is selected from the group consisting of: chitosan and its derivatives, PAA-MA (poly-(acrylic acid-*co*-maleic acid)), also known as poly-(acrylamide-*co*-maleic acid), Poly(4-styrenesulfonic acid-*co*-maleic acid) sodium salt, quaternized chitosan. The pharmaceutically acceptable salts of these compounds are also included in the present invention.

In a first preferred embodiment of the present invention, the polymer is chitosan. Chitosan (Chitosan, Low molecular weight, 75-85% deacetylated, other names: Deacetylated chitin, Poly(D-glucosamine); No. 448869, Aldrich; used in medicine to reduce scars in wound healing) has a pK of around 6.5.

In a second preferred embodiment the polymer is PAA-MA (poly-(acrylic acid-co-maleic acid), also known as poly-acrylamide-co-maleic acid.

They have FDA permission and are known in literature for medical applications. Via water bond and weak electrostatic interactions the partially charged polymer will "wrap" around the cytokine or antibody at pH 7.4. Due to the low number of contact points the interaction will not induce a structural change in the antibody or cytokine molecule which is important to keep them functional. The surfaces of the cytokine and of the antibody are now covered by the polymer therefore they can not be recognized by enzymes like proteases or antibodies which can not detect the antigen and the cytokine. So the molecule construct can travel through the blood without fast clearance and destruction.

If the polymer is now exposed to an acidic environment like found in solid tumors it gets protonated and expands due to repulsive forces between the charged groups. While expanding, the cytokines are released and pH increases because the environmental protons are bound to the polymer. This is quite important because the immune system can only work properly at a pH of 7.4 once it is activated by the released cytokines. The now positively charged polymer can attach to the negatively charged tumor cell surfaces in vicinity. Depending on its amount, the polymer only decorates partly the tumor surface or covers completely the tumor. If it only covers parts of the tumor, it will guide the immune response in this direction because it is a foreign material. As a further effect, the present inventors also found that coated cells are dividing more slowly.

In a second preferred embodiment, the polyelectrolyte used is PAA-MA (Poly(acrylic acid-*co*-maleic acid) sodium salt, also known as Poly(acrylamide-*co*-maleic acid), average M_{w} ∼50,000, 1:1 mole ratio of AA:MA; No. 416061, Aldrich) which has a pKa2 of 6.58 (BAJPAI S. K.; JOHNSON S.; Poly(acrylamide-co-maleic acid) hydrogels for removal of Cr(VI) from aqueous solutions, Part 1: Synthesis and swelling characterization. Journal of applied polymer science, 2006, vol. 100, 2759-2769). The closer the pH is to the pK of a substance the more charged monomers of the polyelectrolyte are protonated in a negatively charged polymer. At pH 7.4 a low number of negatively charged groups of the polymer will induce a weak electrostatic binding between polyanion and antibody or cytokine.

In the acidic environment of the tumor the remaining negative charges of PAA-MA will be also protonated and consequently the environmental pH will increase until it reaches the pK of the polyelectrolyte. Due to the uncharging of the initially negatively charged groups of the polyanion the binding between polymer and cytokine or antibody will be destabilized leading to the release of the immune modulators. If there is polyion attachment to the tumor it will be due to water bridges. If not also here the presence of the synthetic molecule will guide an immune response triggered by the release of the cytokines.

The quantity of used cytokines is around 10 times higher than the ED₅₀ for cell culture announced by the distributor. The therapeutic dosage will be determined according to well-known methods and will be finally decided by the physician.

A further object of the present invention is a method for treating cancer by administering an affective amount of the above particles, preferably to a single organ, e.g. the liver of a patient. A preferred embodiment comprises intraportal and intratumoral injection, respectively for chitosan and PAA-MA particles.

A preferred embodiment of the method is the multi-injection of the particles of the invention in a weekly period.

In a preferred embodiment, the particles comprise chitosan, anti-IL-10 Antibody and IFN-γ. In other preferred embodiments the particles comprise PAA-MA, anti-IL-10 Antibody and IL-4 or PAA-MA, anti-IL-10 Antibody and IFN-y.

The polymers according to the present invention are commercially available via common suppliers or can be prepared in the laboratory according to common general knowledge of those of ordinary skill in the art. For example, chitosan is made by deacetylation of chitin, a compound of the crab shell, while PAA-MA is made by a classical polymerization between monomer and starter. Said polymerization can be radical, cationic and anionic (see also Polymer Handbook, J. Brandrup, EH. Immergut, John Wiley & Sons Australia, Limited, last editi*on).*

A further object of the present invention is a pharmaceutical composition comprising an effective amount of the particle of the invention. In a preferred embodiment said pharmaceutical compositions are for intraportal or intratumoral injection, respectively for chitosan and PAA-MA particles for the treatment of liver tumours.

A preferred embodiment is an article of manufacture for the multi-injection of the particle of the invention in a weekly period. An exemplary embodiment of article of manufacture is a package containing a catheter for administration to the tumour site or in the vena porta. Instructions for the use can be included.

In a preferred embodiment, the particles in said pharmaceutical compositions comprise chitosan, anti-IL-10 Antibody and IFN-γ. In other preferred embodiments the particles comprises PAA-MA, anti-IL-10 Antibody and IL-4 or PAA-MA, anti-IL-10 Antibody and IFN-γ.

Particles are prepared by methods known by the skilled in the according to the common general knowledge. For example, the polymer solution is prepared by solving the polymer in water (pH 7). The cytokines and antibody are mixed with same volume of a polymer solution, and eventually incubated.

Conveniently, carbonate ions are incorporated in the polyelectrolyte matrix in order to stabilize the pH at 7.4 in the tumour site for a longer period.

In a preferred embodiment the tumours treated are gastrointestinal tumours, in particular colon and pancreatic cancers.

The following example further illustrates the invention.

### Example

Material and Methods:
Mouse models

Proper evaluation of new antitumor agents and of the biologic response to modified treatment modalities requires adequate animal models with biological behaviour similar to that of humans. To mimic the systemic character of colon and pancreatic cancer with invasive and metastatic growth, such a model should possess the following properties: reproducible growth rates, reliable metastasizing course, and induction and proof of minimal residual disease (MRD).

Both colon and pancreatic cancer are gastrointestinal tumors which develop liver, lung, and bone and brain metastasis via haematological or lymphatic spread even after R0-resection with micro-/macroscopically proven complete tumor resection. For colon cancer this occurs in terms of liver metastasis in about 30-40% of the patients during the first 3 years after the primary operation. For patients with pancreatic cancer the 5 year survival rates even after histologically proven complete resection of the primary tumor is only between 10-35% depending on the primary tumor stage.

Our treatment strategy focuses on the potential biological effect of drugs like cytokines and antibodies at the time of the detection of the tumor, i.e. before the resection of the tumor takes place as well as after R0-resection of cancer when metastasis occurred.

The used mouse models in our laboratory mimic this stage of the patients by inducing tumor growth with a defined amount of tumor cells via (i) the portal vein into the liver and (ii) via local placement of a specific amount of tumor cells under the liver capsule. These mouse models are established in the Laboratory of Molecular Oncology and Immunology (Prof. Dr. Waaga-Gasser) in Wuerzburg, Germany and they were used to proof the efficacy of the therapy on colon and pancreatic cancer.

### Cell lines

For this study we used the Wuerzburg established mouse cell lines and injected them into Balb/c or C57 BL/6 mice. The CT26.WT (derived from Balb/c mouse) colon carcinoma cell line or Panc02 (derived from C57 BL/6 mouse) pancreatic carcinoma cell line was injected into Balb/c or C57 BL/6 mice respectively. Two different models were used via local placement of a specific amount of tumor cells under the liver capsule (5x10³, 5x10⁴ or 5x10⁵ cells/50µl PBS, each lobe of the liver, tumor growth) and the portal vein into the liver (1x10⁵ cells / 100µl PBS, metastatic model). The animals were then injected with a mixture of recombinant human cytokines and an antibody encapsulated into a polyelectrolyte in a concentration slightly higher than the ED₅₀ determined by the supplier 7 days after the injection of the cells via the local route in the tumor / metastasis or via the portal vein.

### Procedure

At day 0 the animals were injected with CT26.WT or Panc02 cells. Seven days after the injection the particles of the invention were administered either locally or intraportally in the mice. Control animals were only injected with the cells.

In preliminary experiments different interleukins (IL-2, IL-4, IL-12(p7O), IFN-gamma) were tested in combination with the antibody for IL-10 and chitosan or PAA-MA for different concentrations. Two different administration routes were chosen. For both polyelectrolytes a intraportal injection was compared to the results in terms of survival time for local (intratumoral) injection.

The mice were analyzed for overall survival. First, the animals were evaluated for tumor growth and metastasis and documented (photographs). Furthermore, liver, lung tissue, spleen, kidneys, lymph nodes, and spinal column of all animals were collected after sacrificing to detect macroscopically the magnitude and frequency of hepatic and pulmonary metastases. An enlargement of the spleen indicates generally an activation of the immune system and was observed and described as well. The other organs were stored at -80°C for further analysis. From these preliminary experiments it was concluded that the intraportal injection is the most promising one for chitosan based coascervates while the intratumoral was better suitable for PAA-MA based mixtures. IL-12 was found to have a negative effect on the tumor growth and animal survival and therefore was not included in further studies. For this reason, IL-12 is excluded from the present invention. The following dilution factors were figured out as most successful ones.

### Protocols and Results

I. Using Chitosan: the animals were administered via *vena porta* with the drug preparation. The animals were 7 days before injected intraportal with 10⁵ CT26.WT (colon cancer) cells.

### Protocols

### Dilution factors:

| | |
|---|---|
| IL-2: c(initial)=10 µg/1 ml | diluted with water: 1:100 |
| | |
| IL-4: c(initial)=10 µg/1 ml | diluted with water: 1:10 |
| IFN-γ c(initial)=10 µg/1 ml | diluted with water: 1:100 |
| | |
| Anti-IL-10 c(initial)=0.5 mg/1 ml | diluted with water: 1:10 |

The chitosan solution was prepared by mixing 2 mg chitosan LMW (low molecular weight) flakes with 11 mL water (pH 7) one day before the experiments. The chitosan will solve to the solubility limit (0.1 mg/ml). There is still a sediment present.

The cytokins and antibody were mixed with same volume of the supernatant of chitosan LMW (low molecular weight) suspension without flakes.

Several different solutions were injected (100 µl) prepared according to the following protocol:

| Protocol | Chitosan | II-10Ab | II-2 | II-4 | IFN-gamma |
|---|---|---|---|---|---|
| | (µl) | (µl) | (µl) | (µl) | (µl) |
| 1 | 22.5 | 20 | 2.5 | 0 | 0 |
| 2 | 22.5 | 20 | 0 | 2.5 | 0 |
| 3 | 22.5 | 20 | 0 | 0 | 2.5 |
| 4 | 25 | 20 | 2.5 | 0 | 2.5 |
| 5 | 25 | 20 | 0 | 2.5 | 2.5 |

### Results

| **Intraportal injection Protocol** | **Cytokine Protocol** | **Weight gain (7-80 days)** | **Liver tumor at day 7** | **Liver tumor at day 80** | **Animals (n)** |
|---|---|---|---|---|---|
| **1** | Chitosan+ α-IL-10 Ab+ IL-2 | 4.5 | +++ | (+) | 2 |
| **2** | Chitosan+ α-IL-10 Ab+ IL-4 | 4 | ++ | 0 | 2 |
| **3** | Chitosan+ α-IL-10 Ab+ IFN-γ | 4.5 | +++ | 0 | 2 |
| .**4** | Chitosan+ α-Il-10 Ab+ IL-2+IFN-γ | 3.5 | +(+) | 0 | 1 |
| **5** | Chitosan+ α-IL-10 Ab+ IL-4+IFN-γ | 3 | 0(?) | 0 | 2 |
| control | none | +0.5 | | +++ | 2 |

| | | | | | |
|---|---|---|---|---|---|
| 0, no tumor; (+), possible tumor; +, minimal tumor; ++, moderate tumor: +++, distinct tumor | | | | | |

### Observations

### Treated animals

No animal in this series died because of tumor at the time of explanation. No animal was cachectic or endstage. The weight of the animals increased without exceptions. No animal suffered of ascites at the time of explantation. Non-hepatic metastases were seldom encountered

### Controls:

Cachexia and ascites were encountered. Animals were endstage at day 25.

They presented massive non-hepatic metastases and massive hepatic metastases.

### Summary

The survival of the treatment group was significantly higher than the control group (80 days vs. 25 days)

Protocol 3 (Chitosan + anti-IL-10 Ab + IFN-gamma) performed best. A distinct tumor amount on day 7 was macroscopically undetectable on day 80

Protocol 1 (Chitosan + anti-IL-10 Ab + IL-2) performed second best. A distinct tumor amount on day 7 was macroscopically almost completely gone.

With protocol 2 (Chitosan + anti-IL-10 Ab + IL-4) a moderate tumor amount on day 7 was macroscopically gone on day 80.

By applying the protocol 4 (Chitosan + anti-IL-10 Ab + IL-2 + IFN-gamma) a minimal tumor amount on day 7 was gone on day 80.
II. Using PAA-MA the animals were administered intratumoral (local) with the drug preparation. The animals were 7 days before injected with 10³ CT26.WT (colon cancer) cells per lope of the liver (local).

### Protocols

### Dilution factors:

| | | |
|---|---|---|
| IL-2: | c(initial)=10 µg/1 ml | diluted with water: 1:80 |
| | | |
| IL-4: | c(initial)=10 µg/1 ml | diluted with water: 1:8 |
| | | |
| IFN-γ: | c(initial)=10 µg/1 ml | diluted with water: 1:80 |
| | | |
| Anti-IL-10: c(initial)=0.5 mg/1 ml diluted with water: 1:8 | | |

The PAA-MA solution was prepared by solving 6 mg of polymer in 1 ml of water (pH 7).

The cytokins and the antibody were mixed with same volume of PAA-MA solution and incubated for 5 min. The mixing of the polymer with single interleukins should prevent that one polymer chain binds several interleukins but wrap around the single protein.

Several different solutions were injected (80 µl) locally, prepared according to the following protocol:

| Protocol | PAA-MA | II-10Ab | II-2 | II-4 | IFN-gamma | |
|---|---|---|---|---|---|---|
| | (µl) | (µl) | (µl) | (µl) | (µl) | |
| 6 | 0 | 40 | 0 | 0 | 0 | **Reference Example** |
| 7 | 40 | 0 | 0 | 0 | 0 | **Reference Example** |
| 8 | 20 | 20 | 0 | 0 | 0 | **Reference Example** |
| 9 | 22.5 | 20 | 2.5 | 0 | 0 | |
| 10 | 22.5 | 20 | 0 | 2.5 | 0 | |
| 11 | 22.5 | 20 | 0 | 0 | 2.5 | |
| 12 | 25 | 20 | 0 | 2.5 | 2.5 | |
| 13 | 27.5 | 20 | 2.5 | 2.5 | 2.5 | |

### Results

| **Intraportal injection Protocol** | **Cytokine Protocol** | **Weight gain (7-80 days)** | **Liver tumor at day 7** | **Liver tumor at day 80** | **Animals (n)** |
|---|---|---|---|---|---|
| **6** | α-IL-10 Ab | 45 | (+) | (+) | 2 |
| **7** | PAA-MA | 7 | + | (+) | 2 |
| **8** | PAA-MA+ α-1L-10 Ab | 4.5 | ++ | 0 | 2 |
| **9** | PAA-MA+ α-IL-10 Ab+ IL-2 | 35 | + | | 2 |
| **10** | PAA-MA+ α-IL-10 Ab+ IL-4 | 4 | ++ | 0 | 2 |
| **11** | PAA-MA+ α-IL-10 Ab+ IFN-γ | 5.5 | ++ | 0 | 2 |
| **12** | PAA-MA+ α-IL-10 Ab+ IL-4+ IFN-γ | 3 | (+) | 0 | 2 |
| **13** | PAA-MA+ α-IL-10 Ab+ IL-2+IL-4+ IFN-γ | 3.5 | (+) | 0 | 2 |
| control | none | +3.0 | | +++ | 1 |

| | | | | | |
|---|---|---|---|---|---|
| 0. no tumor: (+). possible tumor. +. minimal tumor. ++. moderate tumor: +++. distinct tumor | | | | | |

### Observations

### Treated animals:

No animal in this series died tumor-related. No animal suffered from cachexia or was endstage at the time of explanation. No animal suffered from ascites. The weight increased without exceptions. Non-hepatic metastases were not encountered.

### Controls:

Animals were endstage at day 22.

Massive ascites was encountered.

The liver was completely riddled with tumor.

Numerous non-hepatic metastases were encountered.

### Summary

The survival of the treatment group was significantly higher than the control group (80 days vs. 22 days).

Within the treatment group the protocols 10 (PAA-MA + anti-IL-10 Ab + IL-4) and 11 (PAA-MA + anti-IL-10 Ab + IFN-gamma) performed best. In both cases there were moderate tumor masses on day 7, whereas there was no macroscopic tumor on day 80.

The protocols 7 (PAA-MA only), 8 (PAA-MA + anti-IL-10 Ab), 9 (PAA-MA + anti-IL-10 Ab + IL-2) performed second best, reducing either a moderate tumor mass to a minimal tumor mass or a minimal tumor mass to a macroscopically hardly diagnosable tumor mass.

The protocols 12 and 13 are hard to evaluate, since there was only a minimal tumor mass on day 7. On day 80 there was no macroscopic tumor any more. The application of anti-IL-10 Ab without any PAA-MA did not reduce the tumor mass.

## Claims

1. A particle comprising:
at least one immune-activating cytokine;
at least one antibody against an immune-suppressor cytokine and
a polymer with a pK between 6.5 and 7.4 or a pharmaceutically acceptable salt thereof.

2. The particle of claim 1, wherein the polymer is selected from the group consisting of: chitosan and its derivatives, poly-(acrylic acid-*co*-maleic acid) (PAA-MA), poly(4-styrenesulfonic acid-*co*-maleic acid) sodium salt and quaternized chitosan.

3. The particle of claim of any one of claims 1-2 wherein said immune-activating cytokine is selected from the group consisting of: IL-2, IL-4 and IFN-γ.

4. The particle of any one of claims 1-3 wherein said antibody is Anti-IL-10.

5. The particle of any one of claims 1-4 for use as medicament.

6. The particle of claim 5 for use as drug delivery system, wherein said drug is said at least one immune-activating cytokine and said at least one antibody against an immune-suppressor cytokine.

7. The particle of claim 6 wherein said drug is selectively delivered to the acidic tumor site.

8. The particle of any one of claims 5-7 for use as anti-cancer agent.

9. A pharmaceutical composition comprising an effective amount of particle of any one of claims 1-4.
